Europäisches Patentamt

European Patent Office

Office européen des brevets

(11) Publication number: **0 085 237**
**B1**

(12) **EUROPEAN PATENT SPECIFICATION**

(45) Date of publication of patent specification: **30.07.86**

(51) Int. Cl.⁴: **B 01 J 23/66, C 07 D 301/10**

(21) Application number: **82306546.1**

(22) Date of filing: **08.12.82**

(54) Catalysts for the production of alkylene oxides.

(30) Priority: **30.12.81 GB 8139026**
**14.01.82 GB 8201042**

(43) Date of publication of application:
**10.08.83 Bulletin 83/32**

(45) Publication of the grant of the patent:
**30.07.86 Bulletin 86/31**

(84) Designated Contracting States:
**BE DE FR GB IT NL SE**

(56) References cited:
**EP-A-0 003 642**
**EP-A-0 025 963**
**EP-A-0 057 066**
**DE-A-2 712 785**
**GB-A-2 043 481**

(73) Proprietor: **IMPERIAL CHEMICAL INDUSTRIES PLC**
**Imperial Chemical House Millbank**
**London SW1P 3JF (GB)**

(72) Inventor: **Hayden, Percy**
**2 Hawthorne Drive Hutton Meadows**
**Guisborough Cleveland (GB)**
Inventor: **Johnson, David William**
**559 Yarm Road Eaglescliffe**
**Stockton-on-Tees Cleveland (GB)**

(74) Representative: **Locke, Timothy John et al**
**Imperial Chemical Industries PLC Legal**
**Department: Patents Bessemer Road PO Box 6**
**Welwyn Garden City Hertfordshire, AL7 1HD**
**(GB)**

Courier Press, Leamington Spa, England.

# 0 085 237

## Description

This invention relates to catalysts for the production of alkylene oxides, for example propylene oxide and preferably ethylene oxide.

Catalysts for the production of olefin oxides, for example ethylene oxide by the oxidation of olefines normally comprise silver supported on a heat resisting support. The catalyst supports normally comprise particles of alpha alumina which are fused and/or bonded by cements to form porous pellets into which the silver is introduced for example by impregnation of the support with a solution of a decomposable silver compound, drying and decomposing the silver compound to silver.

We believe that the portions of the support surface which are not covered with silver may possess a catalytic activity which contributes to the total oxidation of the olefine and/or olefine oxide to carbon dioxide and water; for example they may isomerise ethylene oxide to acetaldehyde which is then subject to further oxidation.

It has been proposed (for example in DE—A—2712785, EP—A—3642 and EP—A—57066) to promote the catalysts by the addition of alkali metals, especially cesium and/or rubidium, to them. This has been carried out by impregnating the catalyst or its support before, during or after the introduction of the silver with a solution comprising the alkali metal, the alkali metal being deposited by evaporating the solution. The alkali metal is believed to be present in such catalysts largely in the form of a physical deposit.

We have found that improved catalysts may be obtained by chemically absorbing cesium and/or rubidium on to the surface in a concentration of at least 0.003 and preferably at least 0.008 gram equivalents per kilogram of the element based on the total weight of the catalyst and preferably in an amount equivalent to at least 0.003 gram equivalents of the element per kilogram based on the total weight of the catalyst per square metre per gram of support surface area.

The invention therefore comprises a catalyst for the production of an alkylene oxide which is preferably ethylene oxide by the oxidation of an alkene with oxygen, which comprises silver on a porous heat resisting support which comprises at least 0.003 gram equivalents and preferably at least 0.008 gram equivalents and preferably at most 0.04 and more preferably at most 0.016 gram equivalents of cesium and/or rubidium chemically absorbed on the surface of the support per kilogram of the total catalyst. The amount of cesium and/or rubidium chemically absorbed is suitably in the range 400 to 3000 and preferably 600 to 1500 parts per million per square metre per gram of support surface area. By parts per million is meant parts per million by weight cesium and/or rubidium expressed as the element based on the total weight of the catalyst.

The quantity of chemically absorbed cesium and/or rubidium may be determined as follows. The catalyst may be impregnated with a solution comprising a cesium and/or rubidium salt, drained and dried and analysed for cesium and/or rubidium. The amount of cesium and/or rubidium deposited in the catalyst as a mere result of evaporation of the solution may be calculated from the concentration of the residual solution and the porosity of the catalyst. This gives the cesium and/or rubidium content of the solution contained in the pores of the catalyst. The excess cesium and/or rubidium over and above this figure is the chemically absorbed cesium and/or rubidium. An alternative method of determining the chemically absorbed cesium and/or rubidium is to wash the catalyst with methanol, which removes only physically absorbed cesium and/or rubidium and then with water which removes the more strongly held chemically absorbed cesium and/or rubidium. The quantity of cesium and/or rubidium removed in the second step is that chemically absorbed.

The quantity of chemically absorbed cesium and/or rubidium may be increased by the following means.

(1) Prolonged exposure of the catalyst and/or support to the impregnating solution tends to produce an increase in the level of chemically absorbed cesium and/or rubidium. Typically impregnation should be continued for at least 4 preferably at least 8 and more preferably at least 16 hours.

(2) The level of chemically absorbed cesium and/or rubidium may be increased by impregnating with a salt of cesium and/or rubidium which has a small dibasic anion, for example oxalate or carbonate of which the corresponding acid preferably has a first dissociation constant which is less than 0.1. Salts of strong mineral acids such as nitric and/or sulphuric acids are less suitable because they tend to retain the cesium and/or rubidium in the solution and/or to leave deposits of unwanted elements in the catalyst.

(3) Chemical absorption may also be increased by impregnating with a solution which comprises a lower alcohol for example ethanol and preferably methanol together with a small quantity of water sufficient to dissolve the desired amount of the cesium and/or rubidium compound. Typically the solution comprises 90% v/v or more, preferably at least 95% v/v of the lower alcohol, and the amount of water is preferably the minimum necessary to dissolve the cesium and/or rubidium compound.

(4) The amount of chemically absorbed cesium and/or rubidium may also be increased by using a catalyst support of which the surface comprises a large number of acidic sites, for example at least 0.003 and preferably at least 0.008 gram equivalents per square meter of support surface area. It is believed that the chemical absorption preferably proceeds at least in part by an ion exchange mechanism and that such sites are favourable for this process. Suitably the surface of the catalyst comprises silica and alumina and compounds thereof, the ratio of silica to alumina expressed as atoms of silicon to atoms of aluminium being preferably in the range 1 to 10 to 10 to 1 and more preferably 1 to 3 to 3 to 1. These ratios need apply

2

only on the surface of the support; the bulk of the support may if desired be alpha alumina, silica, an alumino silicate, silicon carbide, zirconium or any other desired substance. Suitably however it is composed of an aggregate of alpha-alumina particles which may be fused together or cemented together with for example silica or baryta.

In order to increase the number of acid sites available for cesium and/or rubidium absorption the surface of the support is preferably cleaned from contaminants before the cesium and/or rubidium is introduced. If other ions, for example alkali metal ions, or basic material, for example amines left on the catalyst from a silver impregnation and decomposition process are present, they may be at least partly removed by washing with a suitable solvent, for example water. If organic material is deposited in the catalyst and impedes chemical absorption of the cesium and/or rubidium it may be removed by oxidation for example with oxygen at elevated temperatures for example 200 to 300°C. As oxidation may affect the surface of the silver present and alter its properties as regards its uptake of cesium and/or rubidium it is preferred that it should be reduced with a reducing agent before a cesium and/or rubidium impregnation step. The reducing agent may be for example hydrogen, but formaldehyde is preferred. This may be present in an aqueous solution used to impregnate the catalyst; the catalyst is then heated, thus reducing the silver and evaporating the water. The formaldehyde is removed in the process leaving a clean surface on which metallic silver particles are present.

The porous heat resisting support preferably has a specific surface area in the range 0.05 to 10 m²/g and preferably 1.0 to 5 m²/g as measured by the Brunauer Emmett and Teller method.

The catalyst support preferably has an apparent porosity as measured by the mercury absorption method of at least 20%, for example 25—80% preferably 25—60% and more preferably 45—60% and mean pore diameters of 0.1 to 20 microns preferably 0.2 to 2 microns as measured by the mercury porosimetry method.

Silver may be introduced to a pre-formed porous heat resisting support as a suspension of silver or silver oxide in a liquid medium for example water or by impregnation of the support with a solution of a silver compound which can be reduced to silver metal if necessary by means of a reducing agent for example hydrogen. If necessary a heat treatment may be used to decompose the silver compound to silver. Suitably the impregnating solution contains a reducing agent which may be for example an anion, for example a formate, acetate, propionate, lactate, tartarate or preferably oxalate ion, of a silver compound in the solution. The reducing agent may be for example an aldehyde, for example formaldehyde or acetaldehyde or an alcohol preferably having 1 to 4 carbon atoms for example methanol or ethanol.

The solution of the silver compound may be a solution in water and/or an organic solvent, for example an aliphatic alcohol preferably having 1 to 4 carbon atoms, a polyhydric alcohol for example ethylene glycol or glycerol, a ketone for example acetone, an ether for example dioxan or tetrahydrofuran, a carboxylic acid for example acetic acid, or molten lactic acid which is preferably used in the presence of water, or an ester for example ethyl acetate or a nitrogen containing base for example pyridine or formamide. An organic solvent may function as a reducing agent and/or complexing agent for the silver also.

If the silver is introduced by impregnating a support with a solution of a decomposable silver compound it is preferred that ammonia and/or a nitrogen containing base should be present. The nitrogen containing base suitably acts as a ligand maintaining the silver in solution; for example it may be pyridine, acetonitrile, an amine, especially a primary or secondary amine having 1—6 carbon atoms, or preferably ammonia. Other suitable nitrogen-containing bases include acrylonitrile, hydroxylamine and alkanolamines for example ethanolamine, alkylene diamines having from 2—4 carbon atoms or amides for example formamide or dimethyl formamide. The nitrogen-containing bases may be used alone or in admixture, mixtures of ammonia and a second nitrogen containing base being preferred. Suitably the nitrogen containing base or bases are used together with water. Very suitably the solution comprises silver nitrate and a lower alkyl amine having 1 or 5 carbon atoms, for example isopropylamine, in water.

Alternatively the solution may be a neutral or acid solution for example it may be a solution of a silver carboxylate especially a formate, acetate, propionate, oxalate, citrate, tartarate or preferably lactate or for example a solution of silver nitrate.

The solutions preferably contain 3—50% of silver by weight.

Impregnation may be carried out in a single stage or if desired may be repeated one or more times. By this means higher silver contents of the catalyst may be achieved.

The silver compound may generally be reduced to silver by heating in the range 100 to 350°C, for example for a period of 15 mins to 24 hours, preferably in the substantial absence of oxygen, for example in the presence of an inert gas for example nitrogen.

Most of the silver content of the catalyst is preferably present in the form of discrete particles adhering to the support having equivalent diameters of less than 10,000 Å (1000 nm) preferably in the range 20—10,000 Å (2—1000 nm) and more preferably 40—8,000 Å (4—800 nm). By equivalent diameter is meant the diameter of a sphere of the same silver content as the particle.

Preferably at least 80% of the silver is present as particles having equivalent diameters in the aforesaid range, the quantity of silver being judged in terms of the number of particles falling in that range. The silver may be present as silver and/or silver oxide and is thought to be present normally as silver particles having a surface layer of silver oxide. The dimensions of the silver particles may be determined by scanning electron microscopy.

The catalyst preferably comprises 3 to 50% and more preferably 5 to 15% by weight of silver.

The rubidium and/or cesium may be introduced to the support before during or after impregnation with a solution of the silver compound but in order to avoid loss of the rubidium and/or cesium or its redeposition on less favoured sites during the impregnation with the silver compound it is preferred to introduce the rubidium and/or cesium after the impregnation and, to avoid redeposition of the silver compound, preferably after decomposition of the silver compound to silver metal. The promoters are suitably introduced as solutions of compounds of the promoting elements, which solutions may be in water and/or organic solvents. If it is desired to impregnate a catalyst which has already been used in the oxidation of an alkene to an alkylene oxide this may be carried out also, whether or not the catalyst already contains one or more promoters.

Any cesium and/or rubidium present as components of the support in non water extractable form is ignored as it does not contribute to catalysis.

The invention also provides processes for the production of alkylene oxides for example ethylene and propylene oxides by the oxidation of the corresponding olefine with oxygen using a catalyst as aforesaid.

Partial pressures of ethylene or propylene in such processes may be in the range 0.1—30 and preferably 1 to 30 bars. The total pressure may be in the range of from 1 to 100 and preferably 3—100 bars absolute. The molar ratio of oxygen to ethylene or propylene may be in the range 0.05 to 100. The partial pressure of oxygen may be in the range 0.01 and preferably 0.1 to 20 bars and preferably 1—10 bars. The oxygen may be supplied for example in the form of air or preferably as commercial oxygen. A diluent for example helium, nitrogen, argon and/or carbon dioxide and/or preferably methane may be present in proportions of 10—80% and preferably 40—70% by volume in total. Ethane may also be present preferably in the range 0.1—5% by volume. It is necessary to operate using gas compositions which are outside the explosive limits.

The temperature is suitably in the range 200—300°C, and preferably in the range 210—290°C. Contact times should be sufficient to convert 0.1—70%, for example·2 to 20 and preferably 5—20% of the ethylene or propylene and unconverted ethylene or propylene is suitably recycled.

A reaction modifier is suitably present. Suitable reaction modifiers comprise chlorine and may be for example chlorinated alkenes having 1—6 carbon atoms for example methyl chloride or tertiary butyl chloride, di-chloromethane or chloroform, a chlorinated biphenyl or polyphenyl, a chlorinated benzene which may be for example monochlorobenzene or·especially vinyl chloride or ethylene dichloride. The concentration of the reaction modifier depends on its chemical nature for example in the case of ethylene dichloride 0.02 to 10 and preferably 0.05—5 parts per million by weight are normally present and in the case of vinyl chloride 0.05—20 and preferably 0.1—10 parts per million by weight are suitably present

We have found that with appropriate concentrations of such reaction modifiers, especially vinyl chloride, attractive selectivities may be secured.

The process may suitable be carried out as described in our co-pending European Patent Application EP—A—57066.

Example 1

Support A of surface area 2.1 m²g⁻¹, pore diameter 0.7 microns and pore volume 0.37 cm³/g composed of α-alumina containing 0.6% silica by weight, where silica was disposed over the surface of the support (measured by X-ray Photoelectron Spectroscopy to have a surface Si:Al ratio of 10%), was used to prepare a silvered catalyst. The silver was added by impregnation of a solution of silver nitrate in aqueous isopropylamine (55 g AgNO₃, 38 g isopropylamine made up to 100 ml with water) onto the support for 20 minutes. After drainage of excess solution the impregnated support was dried for 3 hours at 90°C and then the silver decomposed over 9 hours at temperatures rising from 110°C and 240°C. The drying and pyrolysis stages were carried out in a recirculating nitrogen atmosphere. This catalyst (A) contained 13% silver (w/w).

Catalyst A was impregnated with cesium oxalate in methanol containing 2% v/v water for 4 hours at room temperature. The total volume of solution was three times that needed to fill the pores of the silvered catalyst. The catalyst was dried at 120°C for 1 hour in a forced nitrogen oven. The cesium levels of the final catalyst and the distribution between strongly (i.e. chemically) and weakly absorbed cesium, as determined from the cesium content of the catalyst and the cesium content estimated to be present in the solution in the pores of the catalyst are given in Table 1.

Example 2

Catalyst A was washed with water for 16 hours at 95°C at a liquid hourly space velocity of 4l water per kilogram of catalyst. After drainage it was dried at 180°C for 15 minutes, giving catalyst B.

Catalyst B was impregnated in the same way as Catalyst A in Example 1. The cesium analyses are presented in Table 1. The level of strongly bound cesium is about double that on Catalyst A.

This procedure was repeated except that Catalyst A was washed for different times using the same method. Cesium was then added as in Example 1 the concentration of cesium oxalate in the solution being 1.923 g/litre. The wash time and performance are shown in Table 2.

4

Selectivity determinations

The catalysts were tested for ethylene oxidation activity by passing a gas mixture composed of 30% ethylene, 8% oxygen, 0.6% ethane, 4% carbon dioxide, 0.8 ppm vinyl chloride, 0.8 ppm ethyl chloride, the balance being nitrogen over the catalyst at 16 bar pressure and at a gas hourly space velocity of 3,000 $h^{-1}$. The temperature was adjusted to give a rate of 4 moles ethylene converted per hour per kilogram of catalyst at 40% oxygen conversion.

The selectivity (moles of ethylene oxide produced per 100 moles of ethylene consumed) and the temperatures at which they were obtained are shown in the tables below.

TABLE 1

Catalyst A+Cs

| Cesium oxalate concentration in impregnating solution (g/litre) | Cesium concentration in catalyst | | | | Selectivity/% | Temp/°C |
| --- | --- | --- | --- | --- | --- | --- |
| | Total ppm | Strong | | Weak | | |
| | | ppm | ppm/m²/g | ppm | | |
| 1.398 | 745 | 518 | 246 | 227 | 70.8 | 222 |
| 2.097 | 960 | 540 | 257 | 420 | 71.7 | 232 |
| 2.797 | 1160 | 440 | 210 | 620 | 67.5 | 220 |
| Catalyst B+Cs | | | | | | |
| 1.398 | 910 | 765 | 365 | 145 | 79.2 | 217 |
| 1.748 | 1190 | 1040 | 495 | 155 | 79.0 | 217 |
| 2.097 | 1350 | 1125 | 536 | 225 | 78.0 | 229 |

TABLE 2

| Wash time | Selectivity/% | Temperature/°C |
| --- | --- | --- |
| 0 | 70.8 | 222 |
| 4 | 71.5 | 220 |
| 8 | 74.7 | 215 |
| 16 | 78.2 | 220 |
| 66 | 78.0 | 218 |

ppm means parts per million of cesium by weight based on the total catalyst.

Example 3

Support B of surface area 2.1 m²/g, pore diameter 0.6 micrometre, pore volume 0.30 cm³/g composed of α-alumina containing 0.6% silica by weight (Si:Al surface ratio of 13%) was used to prepare a silver catalyst by the same method as in Example 1. This catalyst, catalyst C was washed for 8 hours at 95°C at a liquid hourly space velocity of 41 water per kilogram of catalyst. After drainage it was dried at 180°C for 15 minutes giving catalyst D.

Catalyst D was impregnated with rubidium carbonate in methanol containing 2% v/v water for 16 hours at room temperature. The volume of solution used was 4.5 times that needed to fill the pores of the support. The concentration of rubidium in the solution was 1.07 g/litre. This is catalyst E. Rubidium analyses are shown in Table 3.

Example 4 (Comparative)

Catalyst D was impregnated with rubidium nitrate in water for 4 hours at room temperature. The volume of solution and concentrations of rubidium were identical to those in Example 3, giving catalyst F. Rubidium analyses are shown in Table 3.

# 0 085 237

TABLE 3

| Catalyst | Rubidium concentration in catalyst | | | |
|---|---|---|---|---|
| | | Strong | | |
| | Total (ppm) | ppm | ppm/m²/g* | Weak (ppm) |
| E | 700 | 435 | 207 | 265 |
| F | 374 | 110 | 52 | 264 |

\* These values are in terms of parts per million per $m^2$ of support surface area per gram of support.

## Claims

1. A catalyst for the production of an alkylene oxide by the oxidation of an alkene with oxygen which comprises silver on a porous heat resisting support which comprises at least 0.003 and preferably 0.008 to 0.016 gram equivalents of cesium and/or rubidium chemically absorbed on the surface of the support per kilogram of the total catalyst by contacting the catalyst with a solution of a compound of cesium and/or rubidium and evaporating the solution, the amount chemically absorbed being assessed by deducting an amount resulting from the evaporation of a quantity of the solution equivalent to that contained in pores of the catalyst from the total amount present.

2. A catalyst as claimed in claim 1 in which the amount of cesium and/or rubidium chemically absorbed is in the range 400—3,000 parts per million of the total catalyst per square metre per gram of support surface area.

3. A catalyst as claimed in claim 1 or 2 in which the catalyst support comprises alpha alumina in which at least 0.008 gram equivalents per square metre of support surface area of acidic sites are present.

4. A catalyst as claimed in claim 1, 2 or 3 in which the porous heat resisting support has a surface area of 1.0 to 5 square metres per gram as measured by a Brunauer Emmett and Teller method, an apparent porosity of 25 to 60% and mean pore diameter of 0.1 to 20 micrometres.

5. A catalyst as claimed in any preceding claim which comprises 5 to 15% by weight of silver.

6. A process for the production of a catalyst as claimed in any preceding claim in which the cesium and/or rubidium is introduced to the catalyst by exposing the catalyst and/or the support to an impregnating solution of a salt of cesium and/or rubidium which has a small dibasic anion and which comprises a lower alcohol together with a small quantity of water sufficient to dissolve the cesium and/or rubidium salt for at least 8 hours.

7. A process for producing a catalyst as claimed in any of claims 1 to 5 in which the rubidium and/or cesium are introduced to a catalyst which comprises silver metal which has been introduced by impregnating a support with a solution of a silver compound, and decomposing the silver compound to silver metal.

8. A process for the production of ethylene oxide which comprises oxidising the ethylene with oxygen in the presence of a catalyst as claimed in any of claims 1 to 5 or produced by a process as claimed in claim 6 or 7 and in the presence of a chlorine containing reaction modifier.

## Patentansprüche

1. Katalysator für die Herstellung eines Alkylenoxids durch die Oxidation eines Alkens mit Sauerstoff, der Silber auf einem porösen, hitzebeständigen Träger enthält, wobei der Träger pro Kilogramm des gesamten Katalysators mindestens 0,003 und vorzugsweise 0,008 bis 0,016 Grammäquivalente Cäsium und/oder Rubidium enthält, das an der Oberfläche des Trägers chemisch absorbiert wird, indem der Katalysator mit einer Lösung einer Verbindung von Cäsium und/oder Rubidium in Berührung gebracht und die Lösung eingedampft wird, wobei die chemisch absorbierte Menge ermittelt wird, indem von der vorhandenen Gesamtmenge eine Menge in Abzug gebracht wird, die sich aus der Eindampfung einer Menge der Lösung, die der in Poren des Katalysators enthaltenen Lösung äquivalent ist, ergibt.

2. Katalysator nach Anspruch 1, bei dem die Menge des chemisch absorbierten Cäsiums und/oder Rubidiums in dem Bereich von 400 bis 3000 Teilen pro Million Teile des gesamten Katalysators und pro Quadratmeter/Gramm spezifische Trägeroberfläche liegt.

3. Katalysator nach Anspruch 1 oder 2, bei dem der Katalysatorträger alpha-Aluminiumoxid enthält, in dem mindestens 0,008 Grammäquivalente saure Stellen pro Quadratmeter Trägeroberfläche vorhanden sind.

4. Katalysator nach Anspruch 1, 2 oder 3, bei dem der poröse, hitzebeständige Träger eine spezifische Oberfläche (gemessen durch die Methode nach Brunauer, Emmett und Teller) von 1,0 bis 5 Quadratmetern

6

pro Gramm, eine scheinbare Porosität von 25 bis 60% und einen mittleren Porendurchmesser von 0,1 bis 20 Mikrometern hat.

5. Katalysator nach einem der vorhergehenden Ansprüche, der 5 bis 15 Gew.-% Silber enthält.

6. Verfahren zur Herstellung eines Katalysators nach einem der vorhergehenden Ansprüche, bei dem das Cäsium und/oder das Rubidium in den Katalysator eingeführt wird, indem der Katalysator und/oder der Träger einer Imprägnierlösung eines Salzes von Cäsium und/oder Rubidium mit einem kleinen zweibasigen Anion, die einen niederen Alkohol zusammen mit einer geringen Wassermenge, die ausreicht, um das Cäsium und/oder das Rubidiumsalz mindestens 8 Stunden lang zu lösen, enthält, ausgesetzt wird.

7. Verfahren zur Herstellung eines Katalysators nach einem der Ansprüche 1 bis 5, bei dem das Rubidium und/oder das Cäsium in einen Katalysator eingeführt wird, der metallisches Silber enthält, das durch Imprägnieren eines Trägers mit einer Lösung einer Silberverbindung und Zersetzung der Silberverbindung zu metallischem Silber eingeführt worden ist.

8. Verfahren zur Herstellung von Ethylenoxid, bei dem das Ethylen in Gegenwart eines Katalysators nach einem der Ansprüche 1 bis 5 oder eines durch ein Verfahren nach Anspruch 6 oder 7 hergestellten Katalysators und in Gegenwart eines chlorhaltigen Reaktions-Modifikationsmittels mit Sauerstoff oxidiert wird.

**Revendications**

1. Catalyseur pour la production d'un oxyde d'alcoylène par oxydation d'un alcène avec de l'oxygène, qui comprend de l'argent sur un support poreux résistant à la chaleur, qui comprend au moins 0,003 et de préférence de 0,008 à 0,016 équivalent.g de césium et/ou de rubidium absorbés chimiquement sur la surface du support par kg du catalyseur total, par mise du catalyseur en contact avec une solution d'un composé de césium et/ou de rubidium et évaporation de la solution, la quantité chimiquement absorbée étant évaluée par déduction d'une quantité résultant de l'évaporation d'une quantité de la solution équivalente à celle qui est contenue dans les pores du catalyseur, de la quantité totale présente.

2. Catalyseur suivant la revendication 1, caractérisé en ce que la quantité de césium et/ou de rubidium absorbée chimiquement est dans la gamme de 400 à 3000 parties par million du catalyseur total par m² d'aire superficielle du support et par gramme de support.

3. Catalyseur suivant la revendication 1 ou 2, caractérisé en ce que le support du catalyseur comprend de l'alpha-alumine dans laquelle se trouve au moins 0,008 équivalent.g de sites acides par m² de l'aire superficielle du support.

4. Catalyseur suivant la revendication 1, 2 ou 3, caractérisé en ce que le support poreux résistant à la chaleur a une aire superficielle de 1,0 à 5 m² par gramme, telle qu'elle est mesurée par la méthode Brunauer Emmett et Teller, une porosité apparente de 25 à 60% et un diamètre moyen de pore de 0,1 à 20 micromètres.

5. Catalyseur suivant l'une quelconque des revendications 1 à 4, caractérisé en ce qu'il comprend 5 à 15% d'argent.

6. Procédé de production d'un catalyseur suivant l'une quelconque des revendications 1 à 5, caractérisé en ce que l'on introduit le césium et/ou le rubidium dans le catalyseur en exposant celui-ci et/ou le support à une solution d'imprégnation d'un sel de césium et/ou de rubidium qui a un petit anion dibasique et qui comprend un alcool inférieur avec une petite quantité d'eau suffisante pour dissoudre le sel de césium et/ou de rubidium, pendant au moins 8 heures.

7. Procédé de production d'un catalyseur suivant l'une quelconque des revendications 1 à 5, caractérisé en ce que l'on introduit le rubidium et/ou le césium dans un catalyseur qui comprend de l'argent métallique, qui a été introduit par imprégnation d'un support avec une solution d'un composé d'argent et décomposition du composé d'argent en argent métallique.

8. Procédé de production d'oxyde d'éthylène qui comprend l'oxydation de l'éthylène avec de l'oxygène, en présence d'un catalyseur suivant l'une quelconque des revendications 1 à 5, ou produit suivant le procédé selon la revendication 6 ou 7 et en présence d'un agent modifiant la réaction contenant du chlore.